(19) Europäisches Patentamt
European Patent Office
Office européen des brevets

(11) Veröffentlichungsnummer: **0 314 011**
**A2**

(12) # EUROPÄISCHE PATENTANMELDUNG

(21) Anmeldenummer: 88117572.3

(22) Anmeldetag: 21.10.88

(51) Int. Cl.[4]: **C07H 19/16 , A61K 31/70**

(30) Priorität: 30.10.87 GB 8725466
13.06.88 GB 8816612

(43) Veröffentlichungstag der Anmeldung:
03.05.89 Patentblatt 89/18

(84) Benannte Vertragsstaaten:
AT BE CH DE ES FR GB GR IT LI LU NL SE

(71) Anmelder: F. HOFFMANN-LA ROCHE & CO. Aktiengesellschaft

CH-4002 Basel(CH)

(72) Erfinder: Lambert, Robert Wilson
33 New Road Digswell
Welwyn Herts.(GB)
Erfinder: Martin, Joseph Amstrong
10 The Chownes West Common
Harpenden Herts.(GB)

(74) Vertreter: Lederer, Franz, Dr. et al
Patentanwält Dr. Franz Lederer Lucile
Grahnstrasse 22
D-8000 München 80(DE)

(54) **Purinderivate.**

(57) Verbindungen der allgemeinen Formel

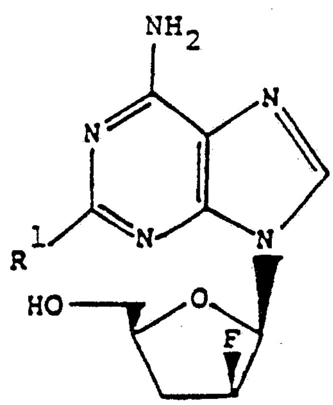

I

worin $R^1$ Chlor, Azido oder Amino ist, sowie Amide und Schiff'sche Basen davon haben antivirale Wirksamkeit und können für die Behandlung oder Prophylaxe von humanen oder veterinären viralen Infektionen insbesondere von retroviralen Infektionen wie HIV verwendet werden.

EP 0 314 011 A2

## Purinderivate

Die vorliegende Erfindung betrifft Purinderivate der allgemeinen Formel

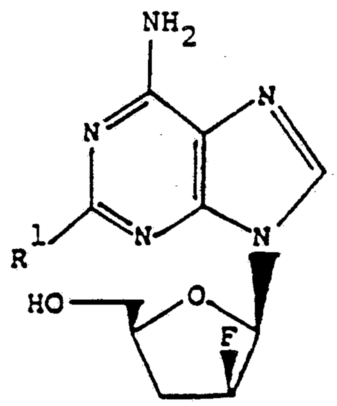

I

worin R[1] Chlor, Azido oder Amino ist, sowie Amide und Schiff'sche Basen davon.

Diese Verbindungen sind neu und haben wertvolle pharmakodynamische Eigenschaften. Insbesondere haben sie antivirale Wirksamkeit und können in der Behandlung und Prophylaxe von viralen Infektionen beim Menschen und beim Tier im speziellen von retroviralen Infektionen wie HIV und dergleichen Verwendung finden.

Gegenstand der vorliegenden Erfindung sind die weiter oben definierten Verbindungen als solche, sowie zur Verwendung als therapeutisch aktive Substanzen, ein Verfahren zur Herstellung dieser Verbindungen, Zwischenprodukte für die Herstellung dieser Verbindungen, Medikamente auf der Basis dieser Verbindungen und die Verwendung dieser Verbindungen zur Behandlung und Prophylaxe von Krankheiten insbesondere von viralen Infektionen, oder bei der Herstellung von Medikamenten für die Behandlung oder Prophylaxe von viralen Infektionen.

Die obigen Verbindungen können in Form von Tautomeren vorliegen die ebenfalls Gegenstand der Erfindung sind.

Besonders bevorzugte Verbindungen der Formel I sind die folgenden:

6-Amino-2-chlor-9-(2,3-dideoxy-2-fluor-β-D-threopentofuranosyl)-9H-purin,
6-Amino-2-azido-9-(2,3-dideoxy-2-fluor-β-D-threopentofuranosyl)-9H-purin und
2,6-Diamino-9-(2,3-dideoxy-2-fluor-β-D-threopentofuranosyl)-9H-purin.

Die obigen Verbindungen können erfindungsgemäss dadurch hergestellt werden, dass man die Gruppe R[2] aus einer Verbindung der allgemeinen Formel

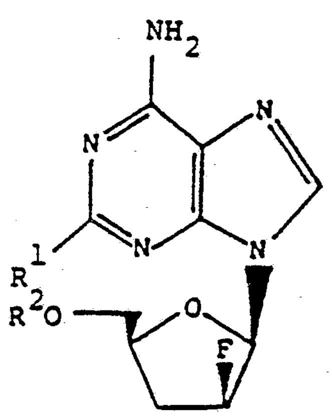

II

worin R' die obige Bedeutung hat und $R^2$ Trityl oder substituiertes Trityl z.B. Monomethoxytrityl ist, abspaltet und gewünschtenfalls eine erhaltene Verbindung der Formel I in ein Amid oder in eine Schiff'sche Base überführt.

Die Abspaltung der Gruppe $R^2$ kann in an sich bekannter Weise erfolgen z.B. durch Behandlung mit einer anorganischen Säure wie Salzsäure in einem Lösungsmittel wie einem halogenierten aliphatischen Kohlenwasserstoff z.B. Chloroform, zweckmässig bei etwa 0 °C bis Raumtemperatur, oder durch Behandlung mit einer starken organischen Säure wie Essigsäure, zweckmässig bei erhöhter Temperatur z.B. bei etwa 100 °C.

Amide der Verbindungen der Formel I werden hergestellt durch Substitution der 6-Aminogruppe und einer allenfalls vorliegenden Aminogruppe $R^1$ durch eine Acylgruppe wie einer Alkanoylgruppe, z.B. Acetyl, Propionyl, Butyryl oder Pivaloyl, oder einer Aroylgruppe z.B. Benzoyl. Schiff'sche Basen der Verbindungen der Formel I werden hergestellt mittels eines Aldehyds oder Ketons wie Benzaldehyd; oder mit einem Dimethylformamidacetal.

Die Verbindungen der Formel II können dadurch hergestellt werden, dass man zuerst eine Verbindung der allgemeinen Formel

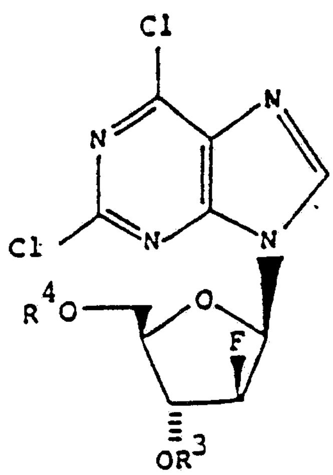

III

worin $R^3$ $C_1$-$C_6$-Alkanoyl, z.B. Acetyl, oder Aroyl, z.B. Benzoyl, und $R^4$ Aroyl ist, mit Ammoniak, zweckmässig mit äthanolischem Ammoniak bei etwa Raumtemperatur während mehreren Tagen, z.B. 7 Tagen behandelt.

Im erhaltenen 6-Amino-2-chlor-9-(2-deoxy-2-fluor-β-D-arabinofuranosyl)-9H-purin der Formel

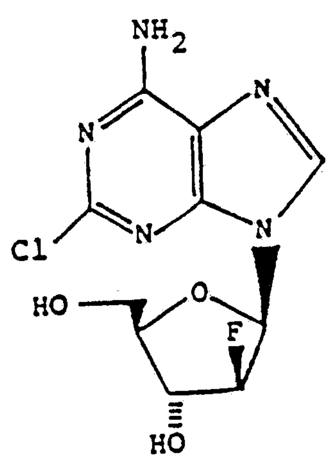

IV

wird dann die 3′-Hydroxygruppe abgespalten. Diese Abspaltung kann in an sich bekannter Weise dadurch erfolgen, dass man zuerst die Verbindung der Formel IV in eine Verbindung der Formel

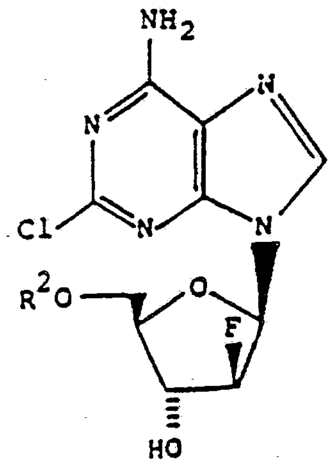

V

worin $R^2$ obige Bedeutung hat, überführt.

Diese Ueberführung kann in an sich bekannter Weise erfolgen mit einem gegebenenfalls substituierten Tritylhalogenid, z.B. Tritylchlorid, in Gegenwart eines säurebindenden Mittels, z.B. eines tertiären Amids wie Pyridin, zweckmässig bei erhöhter Temperatur, z.B. bei etwa 100 °C.

Eine Verbindung der Formel V wird dann mit Phenylthionochloroformat zweckmässig in einem inerten organischen Lösungsmittel wie Acetonitril in Gegenwart eines säurebindenden Mittels wie einem tertiären Amin, z.B. Pyridin oder 4-Dimethylaminopyridin, und bei etwa Raumtemperatur zu einer Verbindung der allgemeinen Formel

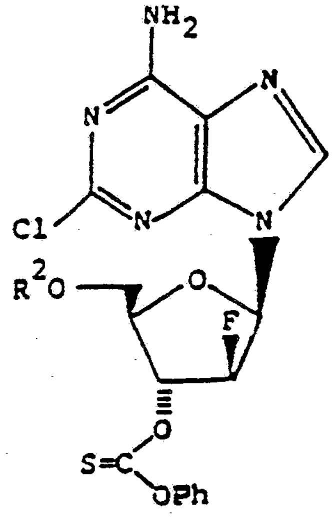

VI

worin $R^2$ die obige Bedeutung hat und Ph Phenyl ist,
umgewandelt.

Eine Verbindung der Formel VI wird dann in eine Verbindung der Formel II übergeführt, in der $R^1$ Chlor ist, durch Behandlung mit Tributylzinnhydrid in Gegenwart eines freien Radikalinitiators. z.B. Azoisobutyronitril, zweckmässig in einem inerten organischen Lösungsmittel wie einem aromatischen Kohlenwasserstoff, z.B. Benzol oder Toluol, und bei erhöhter Temperatur, z.B. bei etwa 60 °-90 °C.

Eine Verbindung der Formel II, in der $R^1$ Chlor ist, kann in eine Verbindung der Formel II, in der $R^1$ Azido oder Amino ist, in an sich bekannter Weise übergeführt werden. So kann das Chloratom $R^1$ durch Azido ersetzt werden durch Reaktion mit Hydrazin und anschliessende Behandlung der entstandenen durch Hydrazino substituierten Verbindung mit einem Alkalimetallnitrit, z.B. Natriumnitrit, in Gegenwart einer starken organischen Säure wie Essigsäure. Eine Azidogruppe $R^1$ kann in eine Aminogruppe umgewandelt werden durch katalytische Hydrierung in Gegenwart eines Palladiumkatalysators und in einem inerten organischen Lösungsmittel. z.B. einem Alkanol wie Aethanol, zweckmässig bei etwa Raumtemperatur. In einer Variante kann das Chloratom $R^1$ durch eine Aminogruppe ersetzt werden durch Reaktion mit Ammoniak in einem inerten organischen Lösungsmittel wie einem nieder-Alkanol, z.B. Methanol, bei erhöhter Temperatur, z.B. bei etwa 140 °C in einem Bombenrohr.

Die Verbindungen der Formel III sind bekannt oder sind wie bekannte Analoga herstellbar.

Die Verbindungen der Formeln II, IV, V und VI sind neu und als solche Gegenstand der vorliegenden Erfindung.

Die Aktivität der Purinderivate der vorliegenden Erfindung gegen HIV kann wie folgt nachgewiesen werden.

In diesem Versuch verwendet man HTLV III, gezüchtet in C8166-Zellen (eine menschliche $CD4^+$ T-Lymphoblastenlinie), RPM1 1640 Medium mit Bicarbonatpuffer, Antibiotika und l0%igem fötalem Rinderserum. Eine Zellensuspension wird mit der 10-fachen $TCD_{50}$ des Virus infiziert. Man lässt 90 Minuten bei 37° C adsorbieren. Die Zellen werden mit dem Medium gewaschen. Der Versuch wird in 6 ml Gewebekulturröhrchen durchgeführt. Jedes Röhrchen enthält $2 \times 10^5$ infizierte Zellen in 1,5 ml Medium. Es werden 15 µl einer Lösung der Substanz in wässrigem Medium oder in DMSO zugesetzt. Nach 72 Stunden Inkubation bei 37° C in einer feuchten 5% $CO_2$ enthaltenden Atmosphäre werden die Kulturen zentrifugiert. Ein aliquoter Teil des Ueberstandes wird solubilisiert und einem Antigenabfangversuch unterworfen unter Verwendung eines primären Antiserums mit besonderer Reaktivität gegen virales Protein 24 und eines Meerrettich-Peroxidase-Detektionssystems. Die Farbenerzeugung wird spektrophotometrisch gemessen und in Abhängigkeit der Konzentration der Testsubstanz dargestellt. Die einen 50%igen Schutz erzeugende Konzentration ($IC_{50}$) wird ermittelt. Die Resultate sind in der folgenden Tabelle angegeben:

**Tabelle**

| Produkt des Beispiels No. | $IC_{50}$ (µM) |
|---|---|
| 1 | 1 |
| 2 | 10 |
| 3 | 1 |

Die Puridinderivate der vorliegenden Erfindung können in Form pharmazeutischer Präparate, die diese Verbindungen zusammen mit einem verträglichen pharmazeutischen Träger enthalten, als Medikamente verwendet werden. Diese Präparate können oral, z.B. in Form von Tabletten, Dragées, Hartgelatinekapseln, Weichkapseln, Lösungen, Emulsionen oder Suspen sionen, oder parenteral, z.B. in Form von Injektionslösungen, verabreicht werden.

Die weiter oben erwähnten Träger können pharmazeutisch inerte, anorganische oder organische Materialien sein. Beispiele von Träger für Tabletten, Dragées und Hartkapseln sind Lactose, Maisstärke oder Derivate davon, Talk, Stearinsäure oder Salze davon. Beispiele von Träger für Weichkapseln sind pflanzliche Oele, Wachse, Fette, halbfeste und flüssige Polyole. Beispiele von Träger für die Herstellung von Lösungen oder Sirups sind Wasser, Polyole, Saccharose, Invertzucker und Glucose. Beispiele von Träger für Injektionslösungen sind Wasser, Alkohole, Polyole, Glycerol und pflanzliche Oele.

Die pharmazeutischen Präparate können auch herkömmliche pharmazeutische Hilfsmittel enthalten. wie Konservierungs-, Solubilisierungs-, Stabilisierungs-, Befeuchtungsmittel, Emulgatoren, Süssmittel, Farb- oder Riechstoffe, Salze zur Veränderung des osmotischen Druckes, Puffer, Ueberzugsmittel oder Antioxidantien. Sie können auch andere therapeutisch wertvolle Substanzen enthalten.

Die Dosierung, in der die Purinderivate der vorliegenden Erfindung verabreicht werden können, kann nach oben und unten in Abhängigkeit von Faktoren, wie der einzelnen zu verabreichenden Verbindung, der Schwere der zu behandelnden Erkrankung und dem Zustand des Patienten, variiert werden. Die Purinderivate der vorliegenden Erfindung kann man in einem täglichen Dosierungsbereich von etwa 0,1 bis 20, vorzugsweise 0,2 bis 15, insbesondere etwa 0,4 bis 10 mg/kg Körpergewicht verabreichen.

Diese Purinderivate können in Einzeldosen oder vorzugsweise in mehreren Dosen, z.B. bis zu 6 über den Tag verabreicht werden. Eine Einzeldosis für eine solche Verabreichungsform kann z.B. von etwa 0,5 bis 300, vorzugsweise etwa 1,0 bis 300 und insbesondere etwa 2,0 bis 200 mg Purinderivat enthalten.

Die pharmazeutischen Präparate kann man so herstellen, dass man die obigen Purinderivate, gewünschenfalls zusammen mit anderen therapeutisch wertvollen Substanzen, mit einem verträglichen pharmazeutischen Träger und gewünschtenfalls einem pharmazeutischen Hilfsstoff vermischt und das Gemisch in die gewünschte Verabreichungsform bringt.

## Beispiel 1

Eine Lösung enthaltend 0,39 g 6-Amino-2 chlor-9-(2,3-dideoxy-2-fluor-5-O-trityl-β-D-threopentofuranosyl)-9H-purin in 24 ml trockenem Chloroform wird abgekühlt und mit 2,2 ml einer 0,35 Kochsalzlösung in Chloroform behandelt. Das Gemisch wird 15 Minuten bei 0-5° C, dann 1,5 Stunden bei Raumtemperatur gerührt und anschliessend unter vermindertem Druck eingedampft. Der Rückstand wird mit 20 ml Diäthyläther behandelt und die Suspension wird 30 Minuten gerührt. Der unlösliche weisse Feststoff wird abfiltriert und mit Diäthyläther gewaschen. Man erhält 0,16 g Produkt. Dieses wird in Wasser gelöst und auf einem Kationenaustauscherharz in $H^+$-Form absorbiert. Nach Waschen mit Wasser wird mit 2M Ammoniaklösung eluiert. Nach Abdampfen des Eluats und Umkristallisation aus Wasser erhält man reines 6-Amino-2-chlor-9-(2,3-dideoxy-2-fluor-β-D-threopentofuranosyl)-9H-purin, Smp. 210-211° C.

Das Ausgangsmaterial wird wie folgt hergestellt:

a) Eine Lösung von 1,96 g 2,6-Dichlor-9-(3-O-acetyl-5-O-benzoyl-2-deoxy-2-fluor-β-D-arabinofuranosyl)-9H-purin in 195 ml Aethanol wird bei -5° C mit gesättigtem Ammoniak gesättigt und dann 7 Tage bei Raumtemperatur gehalten. Die Lösung wird unter vermindertem Druck eingedampft und der Rückstand wird aus 50 ml Aethanol umkristallisiert. Man erhält 0,38 g 6-Amino-2-chlor-9-(2-deoxy-2-fluor-β-D-arabinofuranosyl)-9H-purin, Smp. 212-215° C (Zersetzung). Die Mutterlaugen werden unter vermindertem Druck eingedampft und der Rückstand wird über Silicagel mittels 10% Methanol in Dichlormethan chromatographiert. Man erhält 0,56 g Produkt.

b) Eine Lösung von 0,83 g des Produkts von Beispiel 1a) in 7,5 ml trockenem Pyridin wird bei 100° C unter Argon erhitzt. Eine Lösung von 1,53 g Tritylchlorid in 7,5 ml trockenem Pyridin wird zugetropft. Das Gemisch wird 2 Stunden bei 100° C erhitzt und dann unter vermindertem Druck eingedampft. Der Rückstand wird zwischen 100 ml Wasser und 100 ml Dichlormethan verteilt. Die wässrige Schicht wird abgetrennt und mit Dichlormethan extrahiert. Die organischen Phasen werden über Natriumsulfat getrocknet, filtriert und unter vermindertem Druck eingedampft. Der Rückstand wird über Silicagel mittels 5% Methanol in Dichlormethan chromatographiert. Man erhält 0,84 g 6-Amino-2-chlor-9-(2-deoxy-2-fluor-5-O-trityl -β-D-arabinofuranosyl)-9H-purin, Smp. 210-212° C nach Umkristalliation aus Aethylacetat.

c) Eine Lösung von 0.73 g des Produktes von Beispiel 1b) und 0,365 g 4-Dimethylaminopyridin in 21 ml trockenem Acetonitril wird unter Argon gerührt und mit 0,20 ml Phenylchlorthionocarbonat behandelt. Die Lösung wird über Nacht unter Argon gerührt. Das Lösungsmittel wird unter vermindertem Druck abgedampft und der Rückstand wird über Silicagel mittels 2% Methanol in Dichlormethan chromatographiert. Man erhält 0,72 g 6-Amino-2-chlor-9-(2-deoxy-2-fluor-3-O-Phenoxythiocarbonyl-5-O-trityl-β-D-arabinofuranosyl)-9H-purin in Form eines Schaums.

d) Eine Lösung von 0,72 g des Produktes von Beispiel 1c) und 0,04 g Azobisisobutyronitril in 20 ml trockenem Toluol wird unter Argon gerührt und mit 0,42 g ml Tributylzinnhydrid behandelt. Argon wird 15 Minuten in die Lösung eingeblasen und die Lösung wird dann 2 Stunden unter Argon bei 75° C erhitzt. Das Toluol wird unter vermindertem Druck abgedampft und der Rückstand wird über Silicagel mittels 10% Aceton in Dichlormethan chromatographiert. Man erhält 0,45 g 6-Amino-2-chlor-9-(2,3-dideoxy-2-fluor-5-O-trityl-β-D-threopentofuranosyl)-9H-purin, Smp. 233-234° C nach Umkristallisation aus Diäthyläther.

## Beispiel 2

75 mg 6-Amino-2-azido-9-(2,3-dideoxy-2-fluor-5-O-trityl-β-D-threopentofuranosyl)-9H-purin werden 45 Minuten bei 100° C in 5 ml 80% Essigsäure/Wasser erhitzt. Das Gemisch wird eingedampft und der Rückstand wird mit Toluol eingedampft. Der Rückstand wird über Silicagel mittels Dichlormethan/Methanol (9:1) chromatographiert. Die das Produkt enthaltenden Fraktionen werden eingedampft. Das gummihaltige Produkt wird zu einem Feststoff eingedampft. Dieses wird mit Diäthyläther vermischt. Das Lösungsmittel wird durch Dekantieren entfernt. Der zurückbleibende Feststoff wird im Vakuum getrocknet. Man erhält 20

ml 6-Amino-2-azido-9-(2,3-dideoxy-2-fluor-β-D-threopentofuranosyl)-9H-purin, Smp. 202-204° C (Zersetzung).

Das Ausgangsmaterial wird wie folgt hergestellt:

a) 132 mg 6-Amino-2-chlor-9-(2,3-dideoxy-2-fluor-5-O-trityl-β-D-threopentofuranosyl)-9H-purin (hergestellt wie beschrieben in Beispiel 1d) werden unter Rühren unter Stickstoff 5 ml wasserfreiem Hydrazin zugesetzt. Das resultierende Gemisch wird 2 Tage unter Stickstoff gerührt. 10 ml Wasser werden dann zugesetzt und das Gemisch wird unter Vakuum in Wasser abgedampft. Der Rückstand wird mit Isopropanol unter Hochvakuum eingedampft. Man erhält 6-Amino-2-hydrazino-9-(2,3-dideoxy-2-fluor-5-O-trityl-β-D-threopentofuranosyl)-9H-purin in Form eines weissen Feststoffes.

b) Dem Produkt von Beispiel 2a) gelöst in 15 ml Methanol werden 20 ml Wasser gefolgt von 2 ml Essigsäure zugesetzt. Das Gemisch wird gerührt und 21 mg Natriumhydrid werden zugesetzt. Das Gemisch wird 72 Stunden bei 4° C stehen gelassen, dann eingedampft und zwischen Dichlormethan und Wasser aufgeteilt. Die Dichlormethanphase wird zu einem gummiartigen Feststoff eingedampft. Dieser wird in ein Gemisch von 20 ml Wasser, 30 ml Methanol, 20 ml Dichlormethan und 4 ml Essigsäure gelöst. 69 mg Natriumhydrid werden dann zugesetzt. Man erhält eine Lösung die man 30 Minuten bei Raumtempertur und dann 2 Stunden bei 4° C stehen lässt. Die Lösungsmittel werden eingedampft und der Rückstand wird zwischen Dichlormethan und Wasser aufgeteilt. Die Dichlormethanphase wird über wasserfreiem Natriumsulfat getrocknet und eingedampft. Man erhält ein Feststoff der über Silicagel mittels Dichlormethan/Methanol (9:1) chromatographiert wird. Man erhält 100 mg 6-Amino-2-azido-9-(2,3-dideoxy-2-fluor-5-O-trityl-β-D-threopentofuranosyl)-9H-purin, Smp. 220-222° C.

## Beispiel 3

90 mg 2,6-Diamino-9-(2,3-dideoxy-2-fluor-5-O-trityl-β-D-threopentofuranosyl)-9H-purin werden 1 Stunde bei 100° C mit 10 ml 80% Essigsäure/Wasser erhitzt. Das Gemisch wird im Vakuum eingedampft und der Rückstand wird mit Toluol und dann mit Methanol eingedampft. Der zurückbleibende Feststoff wird in Dichlormethan/Methanol (9:1) gelöst und über Silicagel chromatographiert. Ein Nebenprodukt wird durch Elution mit Dichlormethan/Methanol (9:1) entfernt. Nach Elution mit Dichlormethan/Methanol (4:1) Eindampfen der das erwünschte Produkt enthaltenden Fraktionen und Eindampfen des Rückstands mit Diäthyläther erhält man 30 mg 2,6-Diamino-9-(2,3-dideoxy-2-fluor-β-D-threopentofuranosyl)-9H-purin, Smp. 235-237° C (Zersetzung).

Das Ausgangsmaterial wird wie folgt hergestellt:

Ein Gemisch von 0,131 g 6-Amino-2-chlor-9-(2,3-dideoxy-2-fluor-5-O-trityl-β-D-threopentofuranosyl)-9H-purin, hergestellt wie beschrieben in Beispiel 1d), 7 ml 33% wässriges Ammoniak und 10 ml Methanol wird 40 Stunden bei 140° C gerührt. Das Gemisch wird eingedampft und der entstandene Feststoff wird mit Aethanol und dann mit Methanol eingedampft. Der Rückstand wird in Dichlormethanol/Methanol (9:1) gelöst und über Silicagel mittels Dichlormethan/Methanol (9:1) chromatographiert. Die das Produkt enthaltenden Fraktionen werden eingedampft. Eindampfen des erhaltenen Feststoffs ergibt 90 mg 2,6-Diamino-9-(2,3-dideoxy-2-fluor-5-O-trityl-β-D-threopentofuranosyl)-9H-purin, Smp. 226-230° C (Zersetzung).

Die folgenden Beispiele illustrieren pharmazeutische Zubereitungen mit einem purinderivat nach der vorliegenden Erfindung:

## Beispiel A

Tabletten und Kapseln mit folgenden Inhaltsstoffen können in an sich bekannter Weise hergestellt werden:

| Inhaltsstoffe | | Pro Tablette |
|---|---|---|
| Wirkstoff | | 10 mg |
| Lactose | | 20 mg |
| Stärke | | 4 mg |
| Polyvinylpyrrolidon | | 0,5 mg |
| Magnesiumsteaat | | 0,5 mg |
| | Tablettengewicht | 35 mg |

| Inhaltsstoffe | | Pro Kapsel |
|---|---|---|
| Wirkstoff | | 25 mg |
| Lactose | | 15 mg |
| Natriumstärkeglycollat | | 2,5 mg |
| Magnesiumstearat | | 0,5 mg |
| | Kapselfüllstoff | 43 mg |

## Ansprüche

1. Verbindungen der allgemeinen Formel

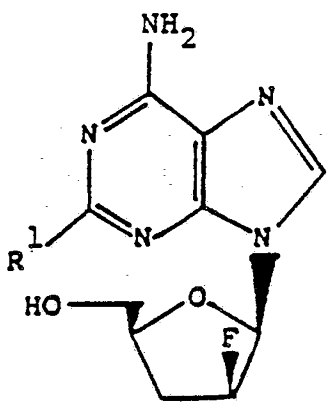

I

worin $R^1$ Chlor, Azido oder Amino ist, sowie Amido und Schiff'sche Basen davon.

2. 6-Amino-2-chlor-9-(2,3-dideoxy-2-fluor-β-D-threopentofuranosyl)-9H-purin.
3. 6-Amino-2-azido-9-(2,3-dideoxy-2-fluor-β-D-threopentofuranosyl)-9H-purin.
4. 2,6-Diamino-9-(2,3-dideoxy-2-fluor-β-D-threopentofuranosyl)-9H-purin.
5. Verbindungen der allgemeinen Formel

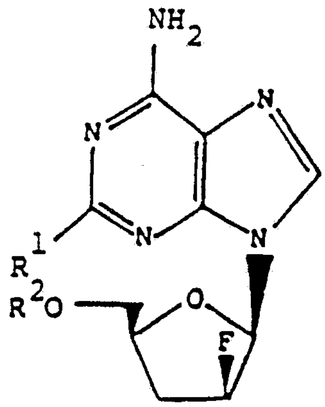

II

worin R¹ Chlor, Azido oder Amino und R² Trityl oder substituiertes Trityl ist.

6. 6-Amino-2-chlor-9-(2-deoxy-2-fluor-β-D-arabinofuranosyl)-9H-purin.

7. Verbindungen der allgemeinen Formel

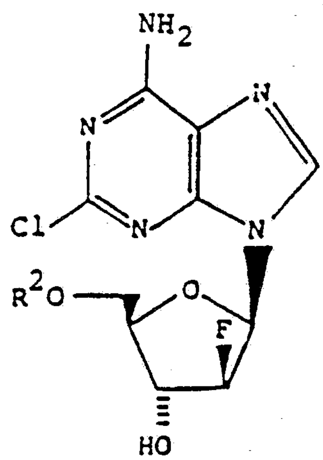

V

worin R² Trityl oder substituiertes Trityl ist.

8. Verbindungen der allgemeinen Formel

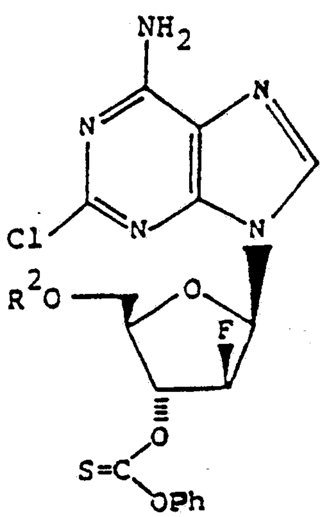

VI

worin R² Trityl oder substituiertes Trityl und Ph Phenyl ist.

9. Purinderivate nach einem der Ansprüche 1-4 zur Anwendung als therapeutisch aktive Substanzen, insbesondere für die Behandlung oder Prophylaxe von viralen Infektionen im speziellen von retroviralen Infektionen und insbesondere von HIV-Infektionen.

10. Verfahren zur Herstellung von Purinderivaten nach einem der Ansprüche 1-4, dadurch gekennzeichnet, dass man die Gruppe $R^2$ aus einer Verbindung der allgemeinen Formel

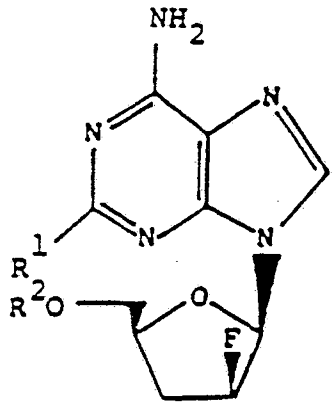

II

worin $R^1$ Chlor, Azido oder Amino und $R^2$ Trityl oder substituiertes Trityl ist, abspaltet und gewünschtenfalls eine Verbindung der Formel I in ein Amid oder eine Schiff'sche Base umwandelt.

11. Ein Medikament, insbesondere zur Behandlung oder Prophylaxe von viralen Infektionen im speziellen von retroviralen Infektionen und insbesondere von HIV-Infektionen auf der Basis von einem Purinderivat nach einem der Ansprüche 1-4.

12. Verwendung eines Purinderivats nach einem der Ansprüche 1-4, bei der Herstellung von Medikamenten für die Behandlung oder Prophylaxe von viralen Infektionen insbesondere von retroviralen Infektionen im speziellen von HIV-Infektionen.